# EUROPEAN PATENT SPECIFICATION

(11) **EP 1 992 611 B1**
(45) Date of publication and mention of the grant of the patent: **07.07.2010**
(21) Application number: 07714368.3
(22) Date of filing: 16.02.2007
(51) Int. Cl.: C07C 271/22, C07K 1/06, C07K 7/04

(54) **ISODIPEPTIDE USEFUL AS A SYNTHETIC UNIT FOR MAKING PEPTIDES**
ISODIPEPTID ALS SYNTHESEEINHEIT ZUR HERSTELLUNG VON PEPTIDEN
ISODIPEPTIDE COMME UNITE DE SYNTHESE UTILE DANS LE SYNTHESE DE PEPTIDES

(30) Priority: 22.02.2006 JP 2006044853
(43) Date of publication of application: 19.11.2008
(73) Proprietor: Kiso, Yoshiaki, Ibaraki-shi, Osaka 567-0827 (JP)
(72) Inventor: Kiso, Yoshiaki, Ibaraki-shi, Osaka 567-0827 (JP)
(74) Representative: Vossius & Partner
(86) International application number: PCT/JP2007/052838
(87) International publication number: WO 2007/097254

(56) References cited:
- YOUHEI SOHMA ET AL: "Novel and efficient synthesis of difficult sequence-containing peptides through O-N intramolecular acyl migration reaction of O-acyl isopeptides" CHEM. COMMUN., no. 1, 2004, pages 124-125, XP002514197
- SOHMA Y ET AL: "'O-Acyl isopeptide method' for the efficient synthesis of difficult sequence-containing peptides: use of 'O-acyl isodipeptide unit'" TETRAHEDRON LETTERS, ELSEVIER, AMSTERDAM, vol. 47, no. 18, 1 May 2006 (2006-05-01), pages 3013-3017, XP025003992 ISSN: 0040-4039 [retrieved on 2006-05-01]
- YOSHIYA T ET AL: "'O-Acyl isopeptide method': racemization-free segment condensation in solid phase peptide synthesis" TETRAHEDRON LETTERS, ELSEVIER, AMSTERDAM, vol. 47, no. 45, 6 November 2006 (2006-11-06), pages 7905-7909, XP025003129 ISSN: 0040-4039 [retrieved on 2006-11-06]
- LORENZ K.B. ET AL.: 'Solution-Phase Synthesis of Nucleotide-Substituted Analogues of Triostin A' JOURNAL OF ORGANIC CHEMISTRY vol. 69, no. 11, 2004, pages 3917 - 3927, XP002319426
- ZEGGAF C. ET AL.: 'Isopropenyl chlorocarbonate (IPCC) in amino acid and peptide chemistry: esterification of N-protected amino acids; application to the synthesis of the depsipeptide valinomycin' TETRAHEDRON vol. 45, no. 16, 1989, pages 5039 - 5050, XP003017314
- OLSEN R.K. ET AL.: 'Synthesis and DNA-binding studies of [lac2,lac6]TANDEM, an analog of des-N-tetramethyltriostin A (TANDEM) having L-lactic acid substituted for each L-alanine residue' JOURNAL OF THE AMERICAN CHEMICAL SOCIETY vol. 108, no. 19, 1986, pages 6032 - 6036, XP003017315
- POZDNEV V.F.: 'Activation of carboxylic acids with pyrocarbonates. Esterification of N-acylamino acids with secondary alcohols using di-tert-butyl pyrocarbonate-pyridine as the condensing reagent' BIOORGANICHESKAYA KHIMIYA vol. 11, no. 6, 1985, pages 725 - 732, XP003017316

## Description

### TECHNICAL FIELD

The present invention relates to a process for preparing a peptide containing an amino acid having a hydroxyl group on its side chain, wherein a specific isodipeptide is used as a synthetic unit. The present invention also relates to a corresponding use.

### BACKGROUND ART

The synthesis of "difficult sequence"-containing peptides is one of the most problematic areas in peptide chemistry. Such peptides are often obtained with significant low yield and purity in conventional solid-phase peptide synthesis. Furthermore, such peptides are generally rich in hydrophobic property and therefore, easily aggregate in various solvents during synthesis and purification. The reason is considered to be due to small aggregates based on β-sheet structure constituted by hydrophobic interaction and hydrogen bond between peptides.

Recently the present inventor et al., have developed "O-acyl isopeptide method" in regard to the effective synthesis of difficult sequence-containing peptides (See non-patent literatures 1 and 2.). This method relates to prepare O-acyl isopeptide by isomerizing amide bond into ester bond, and then to prepare the objective peptide by intramolecular O-N acyl migration, in case of the amino acid containing hydroxyl group such as serine residue.

However, in order to prepare the objective peptide (hereinafter described as a peptide including a long or short chained peptide and a protein) in high yield and high purity by practicing this method, the higher technique is still required and further it is found that on the way of reaction, namely in esterification, for example racemization in esterified Val residue occurs in high frequency and therefore, the decrease of the yield and troublesome purification are accompanied with.
[Non-patent literatures 1] Tetrahedron Letters 45 (2004) 5965-5968
[Non-patent literatures 2] Chem. Commun., 2004, 124-125

Tetrahedron Letters, 2006, 47(18): 3013-3017, which has been published in the priority interval of the present application, relates to an 'O-acyl isodipeptide unit', Boc-Thr(Fmoc-Val)-OH, and its use for the synthesis of a difficult sequence-containing pentapeptide based on the 'O-acyl isopeptide method', in which racemization-inducible esterification can reportedly be omitted.

Tetrahedron Letters, 2006, 47(45): 7905-7909, which has been published in the priority interval of the present application, relates to a 'racemization-free segment condensation' based on the 'O-acyl isopeptide method' in which an N-segment including C-terminal O-acyl isopeptide structure with urethane-protected Ser/Thr residue is employed for the segment condensation.

### DISCLOSURE OF INVENTION

The present inventor has earnestly investigated to solve the above problems and has found that by using an isodipeptide (1) described below, especially as a synthetic unit in solid-phase peptide synthesis, the objective peptide can be easily prepared in high yield and high purity. Thus the present invention was completed.

The present invention relates to the use of an isodipeptide of the following formula (1) as a synthetic unit in the preparation of a peptide containing an amino acid having hydroxy group on its side chain, and a process for preparing the peptide wherein the isodipeptide of Formula (1) is used : 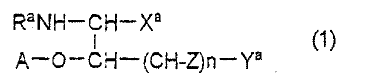 wherein A is N-protected amino acid residue, R^{a} is amino protective group, X^{a} is carboxyl group, hydrogen atom, alkyl group, aralkyl group, aryl group or heteroaryl group, Y^{a} is carboxyl group, hydrogen atom or alkyl group, Z is hydrogen atom or alkyl group, and n is an integer of 0-3, provided that either one of X^{a} and Y^{a} is carboxyl group.

### Effect of invention

In the solid-phase peptide synthesis by using isodipeptide synthetic unit of the present invention, the desired peptide can be fully automatically synthesized and therefore, its synthesis is very easy and the present invention is very valuable from a viewpoint of the industrial application.

Furthermore, by using the isodipeptide (1) of the present invention as a synthetic unit for a peptide, it is unexpectedly found to avoid the above mentioned side reaction such as racemization and to obtain the objective peptide in high yield and purity.

### MODE FOR CARRYING OUT THE INVENTION

The present invention relates to the use of the isodipeptide represented by the following formula (1) as a synthetic unit in the preparation of a peptide containing an amino acid having hydroxy group on its side chain, and a process for preparing the peptide wherein the isodipeptide of Formula (1) is used : 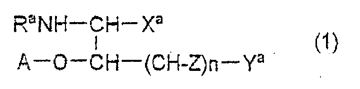 wherein A, R^{a}, X^{a}, Y^{a} and n are the same as defined above.
Alkyl group in X^{a} and Y^{a} of the formula (1) is not limited, but is preferably C₁₋₆ straight or branched chain alkyl group. Aryl or heteroaryl moiety of aralkyl group, aryl group or heteroaryl group in X^{a} of the formula (1) may be substituted by a substituent such as methyl, nitro, or chlorine atom.

As a preferable isodipeptide in the formula (1) wherein n is 0 or 1, an isodipeptide of the formula (1) wherein X^{a} is carboxyl group, Y^{a} is hydrogen atom or alkyl group, and n is 0, or its optical isomer, and an isodipeptide wherein X^{a} is hydrogen atom, alkyl group, aralkyl group, aryl group or heteroaryl group, Y^{a} is carboxyl group and n is 0, or its optical isomer are illustrated.

As N-protective group of an amino acid or a protective group represented by R^{a} in the formula (1) are illustrated usual protective groups of an amino acid. As preferable protective groups, there are illustrated such protective groups that ester bond in the isodipeptide (1) is not cleaved and only the object protective group is cleaved. For example, urethane type-protective groups, such as 9H-fluoren-9-ylmethoxycarbonyl (Fmoc), tert-butoxycarbonyl (Boc), benzyloxycarbonyl (Z), or 2-chlorobenzyloxycarbonyl, are illustrated.

In the acid residue of N-protected amino acid represented by A, when said amino acid has a functional group such as hydroxy group on its side chain and further carboxyl group, such groups are preferably protected by a suitable known protective group.

The especially preferable compound in the isodipeptide (1) is an isodipeptide of the following formula (1a): 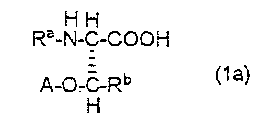 wherein A and R^{a} are the same as defined above, and R^{b} is hydrogen atom or methyl group.

This compound (1a) is especially valuable in the method for synthesizing polypeptides containing L-threonine or L-serine as a component.

The preferable compound is also an isodipeptide of the following compound (1b): 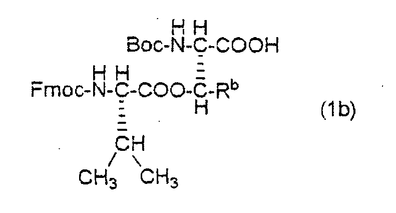 wherein Fmoc is 9H-fluoren-9-ylmethoxycarbonyl, Boc is tert-butoxycarbonyl, and R^{b} is the same as defined above.

This compound (1b) is especially valuable in the method for synthesizing a peptide containing L-valine-L-threonine or L-valine-L-serine as a dipeptide-component.

The most preferable compound in the isodipeptide (1) is the compound of the following formula (1c) (hereinafter sometimes abbreviated as "Boc-Thr(Fmoc-Val)-OH") 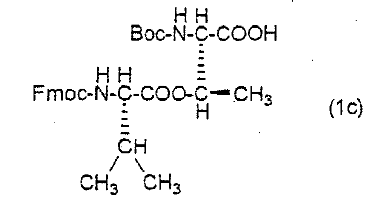 wherein Fmoc and Boc are the same as defined above.

Furthermore, the compounds derived from the isodipeptide (1) of the present invention are obtained by modification of the isodipeptide (1) and having the same function as the compound of the present invention are included in the scope of the present invention as a matter of cause.

The isodipeptide (1) of the present invention can be prepared by known esterification using a carboxylic acid and an alcohol.

Namely an isopeptide (2b) is obtained by reacting a N-protected amino acid represented by A-OH and compound (2a) prepared by protecting carboxyl group of an amino acid having a hydroxyl group on its side chain represented by the following formula (2): 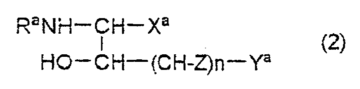 wherein R^{a}, X^{a}, Y^{a}, Z and n are the same as defined above, and then, by deprotecting only the protective group of the carboxyl group of the said compound (2b) to give the isodipeptide (1).

The compounds of the formula (2) preferably include for example, an amino acid having hydroxyl group on its side chain, such as threonine, serine, statine, or norstatine.

N-protected amino acid represented by A-OH is not limited and includes various typed amino acids such as α or β-amino acid having N-protected amino group.

When N-protected amino acid represented by A-OH has a functional side chain, in order to avoid side reaction, the group is preferably protected by the known protecting method.

The above esterification is carried out by the conventional method. The solvent used includes chloroform, or dichloromethane. The reaction temperature depends on the starting materials, but is usually around 25°C.

The protective group of carboxyl group of the compound (2a) preferably includes groups which are removed by hydrogenation such as benzyl group, p-nitrobenzyl group, or 4-pyridylmethyl group in a viewpoint of avoidance of cleavage of the ester part. Furthermore, from this viewpoint the above mentioned amino protective group of isodipeptide (1) preferably includes the protective groups which are not removed by hydrogenation.

The removal of carboxyl group of compound (2b), an intermediate is carried out, for example by introducing hydrogen gas in the presence of Pd/C.

Thus obtained isodipeptide (1) is conventionally isolated, and purified to give the purified product. The purified product is served as a synthetic unit for preparing a desired polypeptide.

A-OH and compound (2) which are starting materials may have one or more asymmetric carbons and therefore, the objective isodipeptide can be obtained in the form of optical isomer, racemic compound thereof, diastereomer or the mixture thereof, depending on the kind of starting materials. For example, by using a respective optical isomer, there is obtainable an isodipeptide in diastereomer type (See (1b) and (1c).).

When the product is obtained in racemic mixture or diastreomer mixture, the product is conventionally optically resoluted and purified along the lines of the object to give a desired isodipeptide with highly optical purity.

Taking a pentapeptide, Ac-Val-Val-Thr-Val-Val-NH₂ (3a) as an example, and using Boc-Thr(Fmoc-Val)-OH (1c) as a synthetic unit, the synthetic scheme in accordance with the solid-phase peptide synthesis is shown below. 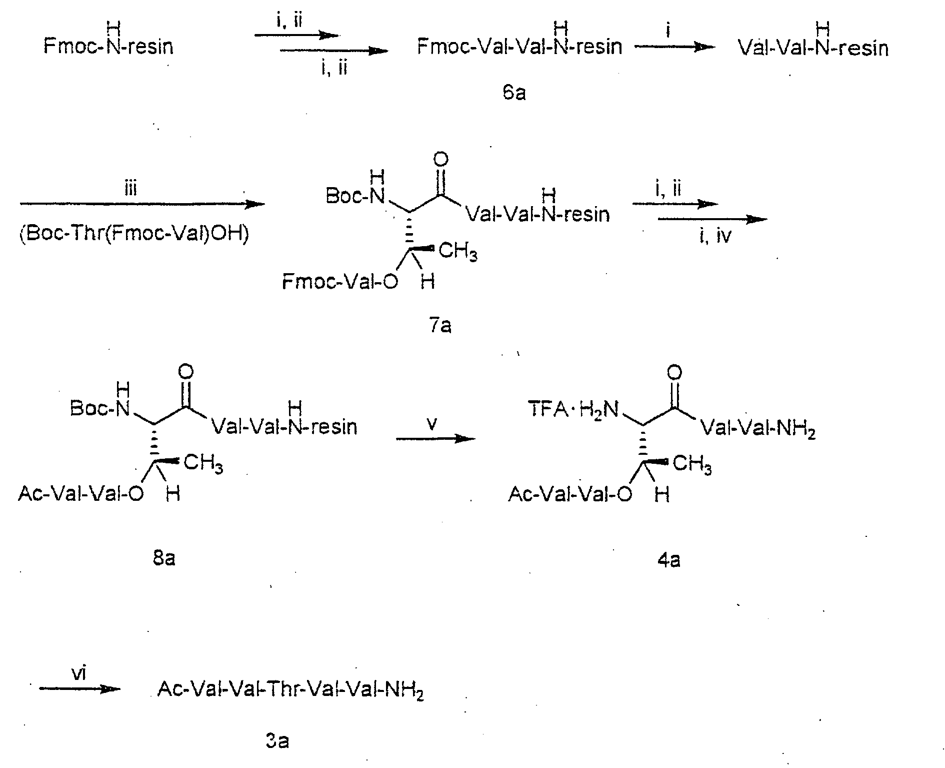 (In the above formulas, Fmoc and Boc are the same as defined above, TFA is tetrafluoroacetic acid and all of the amino acids are L-form.)

The reaction conditions in each step of the above reaction route are as follows:
Reaction i: 20% piperidine/DMF for 20 minutes.
Reaction ii: Fmoc-Val-OH (2.5eq), 1,3-diisopropylcarbodiimide (2.5eq), 1-hydroxybenzotriazole (2.5eq) in DMF for 2 hrs.
Reaction iii: Boc-Thr(Fmoc-Val)OH (2.5eq), 1,3-diisopropylcarbodiimide) (2.5eq), 1-hydroxybenzotriazole (2.5eq) in DMF for 2 hrs.
Reaction iv: Ac₂O (1.5eq), triethylamine (1.0eq) in DMF for 2 hrs.
Reaction v: TFA-m-cresol-thioanisole-H₂O 1.5 hrs.
Reaction vi: phosphate buffer saline solution, pH 7.4 (25°C).

Instead of the above Fmoc-Val-OH, using other amino acid protected by, e.g., Fmoc, there are obtained desired various difficult sequence-containing peptides. Coupling reaction of other amino acid against an amino acid or a peptide is carried out by the conventional method in peptide synthesis. O-N intramolecular acyl migration reaction on ester (Reaction vi) is carried out by the known method (See non-patent literature 1.).

The present invention is further explained by the following examples, but should not be limited by these examples.

### Example 1

### Synthesis of Boc-Thr(Fmoc-Val)-OH

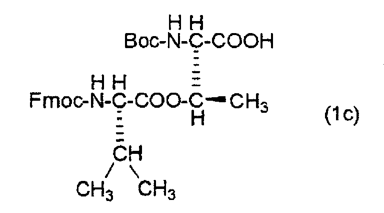

N-(t-Butoxycarbonyl)-L-threonine benzyl ester (Boc-Thr-OBzl) (139mg, 0.449mmol) was dissolved in dry CHCl₃ (10mL), and thereto were added at 0°C N-(9H-fluoren-9-ylmethoxycarbonyl)-L-valine (Fmoc-Val-OH) (183mg, 0.539mmol),4-dimethylaminopyridine (5.5mg, 0.045mmol) and 1-ethyl-3-(3'-dimethylaminopropyl)carbodiimide-HCl (104mg, 0.539mmol), respectively. The reaction mixture was gradually warmed to room temperature in a period of 2 hours and stirred at the same temperature for 5 hours. After diluted with AcOEt, the solution was washed with H₂O, 1M HCl, saturated NaHCO₃ and saturated brine, dried over MgSO₄ and concentrated in vacuo. The resulting residue was purified by silica gel chromatography (AcOEt: hexane 1:4) to give Boc-Thr(Fmoc-Val)-OBzl (266mg, 0.422mmol, yield 94%). In the above reaction racemization was not observed (This was confirmed by separately synthesizing D-valine derivatives.). Then to Boc-Thr(Fmoc-Val)-OBzl (236mg, 0.374mmol) in AcOEt (10mL) was added Pd/C (12 mg) and the mixture was vigorously stirred for 3 hours. After removal of Pd/C through Celite, the solvent was concentrated in vacuo, filtered by silica gel (AcOEt: hexane 1:2) and washed with MeOH to give objective Boc-Thr(Fmoc-Val)-OH (3a) with high purity (186 mg, 0.346mmol, yield 92%).
HRMS (FAB): calcd. for C₂₉H₃₆N₂O₈Na (M+Na)⁺: 563.2369, found: 563.2373; HPLC analysis at 230 nm: purity 95% or more; NMR (CD3OD, 400 MHz): δ 7.79 (d, ³J(H,H) = 7.3 Hz, 2 H, CH), 7.75-7.66 (m, 2H, CH), 7.38 (t, ³J(H,H) = 7.5 Hz, 2 H, CH), 7.33-7.29 (m, 2H, CH), 5.44-5.41 (m, 1H, CH), 4.38 (d, ³J(H,H) = 7.0 Hz, 2 H, CH₂), 4.25-4.22 (m, 2 H, CH), 4.05-4.01 (m, 1 H, CH), 2.11-2.02 (m, 1 H, CH), 1.44 (s, 9 H, CH₃), 1.25 (d, ³J(H,H) = 6.4 Hz, 3 H, CH₃), 0.91, 0.89 (2d, 3J(H,H) = 7.7, 7.0 Hz, 6 H, CH₃).

### Example 2

Instead of Boc-Thr-OBzl, using N-(t-butoxycarbonyl)-L-serine benzyl ester (Boc-Ser-OBzl) in accordance with the method of the above example 1, there can be obtained Boc-Ser(Fmoc-Val)-OH.

### Example 3

### Synthesis of Ac-Val-Val-Thr-Val-Val-NH₂ (3a)

Using Rink Amide AM resin (100mg, 0.071mmol), after washing this resin with DMF (1.5mL×5), according to the sequence by using Fmoc-Val-OH there was constructed H-Val-Val-NH-resin by the conventional method. This product was condensed with Boc-Thr(Fmoc-Val)-OH (100mg, 0.18mmol) which was synthesized by example 1 in DMF (1.5mL) in the presence of 1,3-diisopropylcarbodiimide (29.0µL, 0.18mmol) and 1-hydroxybenzotriazole (28.4mg, 0.18mmol). Then after introducing Fmoc-Val-OH (62.8mg, 0.18mmol) thereto, N-acetylation was carried out by using Ac₂O (10.5µL, 0.11mmol)-triethylamine (10.4µL, 0.071mmol). The protected peptide resin was stirred under the presence of thioanisole (66.7µl), m-cresol (66.7µl) and water (66.7µL) in TFA (2.47mL) for 90 minutes. The reaction solution was concentrated, washed with Et₂O, suspended in water and lyophilized to give O-acyl isopeptide (4a) of the following formula: 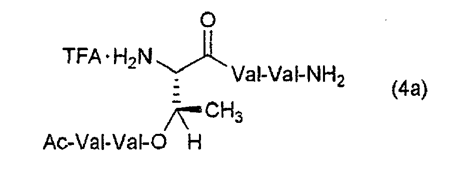 as white amorphous powders (21.2 mg, yield 44.5%).
HRMS (FAB): calcd for C₂₆H₄₉N₆O₇ (M+H)⁺: 557.3663, found: 557.3666; HPLC analysis at 230 nm: purity 95% or more.

As H-Thr-Val-Val-NH₂ was not detected in this reaction products, it is estimated that ester bond formed is stable in piperidine or by TFA treatment, and in case of de-Fmoc reaction in the last Val, diketopiperazine-formation was not proceeded.

The above obtained O-acyl isopeptide (4a) (3.0 mg) was dissolved in phosphate buffer saline solution (pH 7.4) (3mL) and the solution was stirred at room temperature overnight. The resulting white deposite was filtered, washed with H₂O and MeOH, and dried in vacuo to give Ac-Val-Val-Thr-Val-Val-NH₂ (3a) as white powders (yield 2.4 mg (96%)).
HRMS (FAB): calcd. for C₂₆H₄₉N₆O₇ (M+H)⁺: 557.3663, found: 557.3667; HPLC analysis at 230 nm: Purity 95% or more; retention time of the obtained compound on HPLC (0-100% CH₃CN 40 minutes 230nm) was correspond to that of peptide (3a) conventionally synthesized.

Compound (4a) was stable at 4°C at least for 2 years. On the other hand, in case that compound (4a) is dissolved in phosphate buffer saline solution (pH 7.4) and stirred at room temperature, quantitative O-N intramolecular acyl migration to peptide (3a) was confirmed without side reaction.

### INDUSTRIAL APPLICABILITY

By using the isodipeptide (1) of the present invention as a peptide synthesis-unit, the desired peptide can be fully automatically obtained by solid-phase peptide synthesis as well as to avoid side reaction such as racemization.

## Claims

1. A process for preparing a peptide containing an amino acid having hydroxy group on its side chain, wherein an isodipeptide of the following formula (1) is used as its synthetic unit: 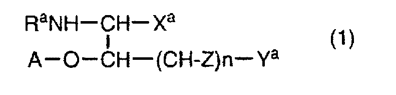 wherein A is N-protected amino acid residue, R^{a} is amino protective group, X^{a} is carboxyl group, hydrogen atom, alkyl group, aralkyl group, aryl group or heteroaryl group, Y^{a} is carboxyl group, hydrogen atom or alkyl group, Z is hydrogen atom or alkyl group, and n is an integer of 0-3, provided that either one of X^{a} and Y^{a} is carboxyl group.

2. The process according to claim 1, wherein n is 0 or 1.

3. The process according to claim 1, wherein in the formula (1) X^{a} is carboxyl group, Y^{a} is hydrogen atom or alkyl group and n is 0.

4. The process according to claim 1, wherein in the formula (1) X^{a} is hydrogen atom, alkyl group, aralkyl group, aryl group, or heteroaryl group, Y^{a} is carboxyl group, and n is 0.

5. The process according to claim 1, wherein the peptide is a peptide containing L-threonine or L-serine as the peptide component and wherein the isodipeptide of the formula (1) is an isodipeptide represented by the following formula (1a): 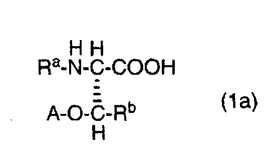 wherein A and R^{a} are the same as defined above, and R^{b} is hydrogen atom or methyl group.

6. The process according to claim 1, wherein the peptide is a peptide containing L-valine-L-threonine or L-valine-L-serine as the peptide component and wherein the isodipeptide of the formula (1) is an isodipeptide represented by the following formula (1b): 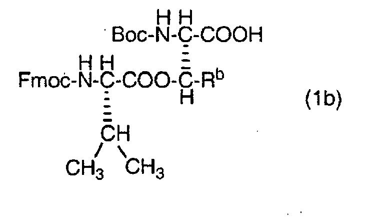 wherein Fmoc is 9H-fluoren-9-ylmethoxycarbonyl group, Boc is tert-butoxycarbonyl group and R^{b} is the same as defined above.

7. The process according to claim 1, wherein the peptide is a peptide containing L-valine-L-threonine or L-valine-L-serine as the peptide component and wherein the isodipeptide of the formula (1) is an isodipeptide represented by the following formula (1c): 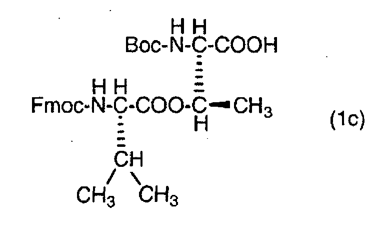 wherein Fmoc and Boc are the same as defined above.

8. Use of an isodipeptide of the formula (1) as defined in any of claims 1 to 4 as a synthetic unit in the preparation of a peptide containing an amino acid having hydroxy group on its side chain.

9. The use according to claim 8, wherein the peptide is a peptide containing L-threonine or L-serine as the peptide component and wherein the isodipeptide of the formula (1) is an isodipeptide represented by the formula (1a)as defined in claim 5.

10. The use according to claim 8, wherein the peptide is a peptide containing L-valine-L-threonine or L-valine-L-serine as the peptide component and wherein the isodipeptide of the formula (1) is an isodipeptide represented by the formula (1b) as defined in claim 6.

11. The use according to claim 8, wherein the peptide is a peptide containing L-valine-L-threonine or L-valine-L-serine as the peptide component and wherein the isodipeptide of the formula (1) is an isodipeptide represented by the formula (1c) as defined in claim 7.

## Patentansprüche

1. Ein Verfahren zur Herstellung eines Peptids, welches eine Aminosäure mit einer Hydroxygruppe an seiner Seitenkette enthält, wobei ein Isodipeptid der folgenden Formel (1) als seine synthetische Einheit verwendet wird: 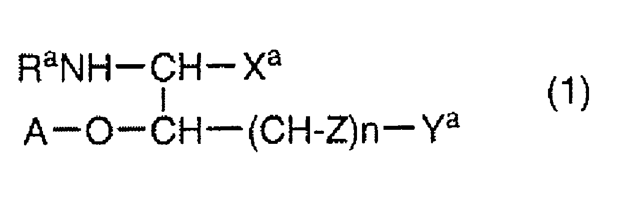 wobei A ein N-geschützter Aminosäurerest ist, R^{a} eine Aminoschutzgruppe ist, X^{a} ein Carboxylrest, ein Wasserstoffatom, ein Alkylrest, ein Aralkylrest, ein Arylrest oder ein Heteroarylrest ist, Y^{a} ein Carboxylrest, ein Wasserstoffatom oder ein Alkylrest ist, Z ein Wasserstoffatom oder ein Alkylrest ist und n eine ganze Zahl von 0 bis 3 ist, mit der Maßgabe, dass entweder X^{a} oder Y^{a} ein Carboxylrest ist.

2. Das Verfahren gemäß Anspruch 1, wobei n für 0 oder 1 steht.

3. Das Verfahren gemäß Anspruch 1, wobei in der Formel (1) X^{a} ein Carboxylrest ist, Y^{a} ein Wasserstoffatom oder ein Alkylrest ist und n für 0 steht.

4. Das Verfahren gemäß Anspruch 1, wobei in der Formel (1), X^{a} ein Wasserstoffatom, ein Alkylrest, ein Aralkylrest, ein Arylrest oder ein Heteroarylrest ist, Y^{a} ein Carboxylrest ist und n für 0 steht.

5. Das Verfahren gemäß Anspruch 1, wobei das Peptid ein Peptid ist, dass L-Threonin oder L-Serin als die Peptidkomponente enthält, und wobei das Isodipeptid der Formel (1) ein Isodipeptid, dargestellt durch die folgende Formel (1a), ist: 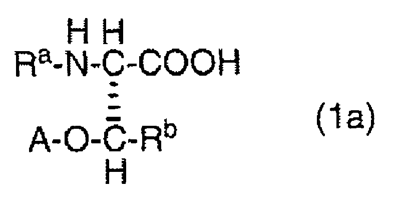 wobei A und R^{a} wie vorstehend definiert sind und R^{b} ein Wasserstoffatom oder eine Methylgruppe ist.

6. Das Verfahren gemäß Anspruch 1, wobei das Peptid ein Peptid ist, dass L-Valin-L-Threonin oder L-Valin-L-Serin als die Peptidkomponente enthält, und wobei das Isodipeptid der Formel (1) ein Isodipeptid, dargestellt durch die folgende Formel (1b), ist: 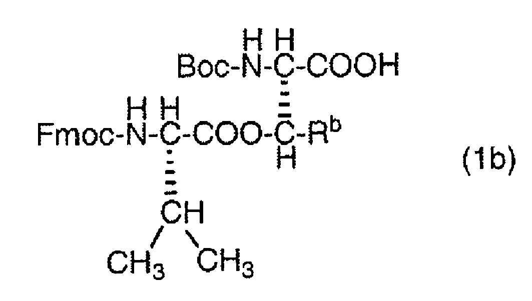 wobei Fmoc ein 9H-Fluoren-9-ylmethoxycarbonylrest ist, Boc ein tert.-Butoxy-carbonylrest ist und R^{b} wie vorstehend definiert ist.

7. Das Verfahren gemäß Anspruch 1, wobei das Peptid ein Peptid ist, dass L-Valin-L-Threonin oder L-Valin-L-Serin als die Peptidkomponente enthält, und wobei das Isodipeptid der Formel (1) ein Isodipeptid, dargestellt durch die folgende Formel (1c), ist: 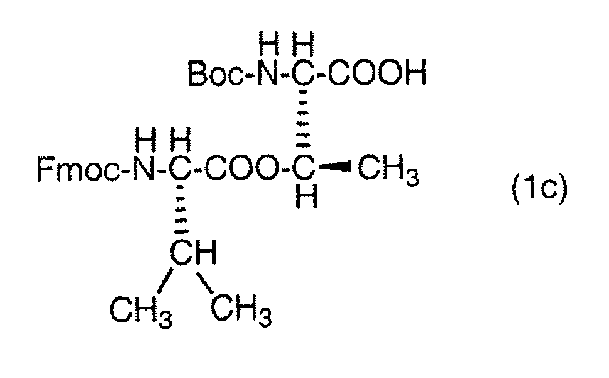 wobei Fmoc und Boc wie vorstehend definiert sind.

8. Verwendung eines Isodipeptids der Formel (1), wie in einem der Ansprüche 1 bis 4 definiert, als eine synthetische Einheit zur Herstellung eines Peptids, welches eine Aminosäure mit einer Hydroxygruppe an seiner Seitenkette enthält.

9. Die Verwendung nach Anspruch 8, wobei das Peptid ein Peptid ist, dass L-Threonin oder L-Serin als die Peptidkomponente enthält, und wobei das Isodipeptid der Formel (1) ein Isodipeptid, dargestellt durch die Formel (1a), wie in Anspruch 5 definiert, ist.

10. Die Verwendung gemäß Anspruch 8, wobei das Peptid ein Peptid ist, dass L-Valin-L-Threonin oder L-Valin-L-Serin als die Peptidkomponente enthält, und wobei das Isodipeptid der Formel (1) ein Isodipeptid, dargestellt durch die Formel (1b), wie in Anspruch 6 definiert, ist.

11. Die Verwendung gemäß Anspruch 8, wobei das Peptid ein Peptid ist, dass L-Valin-L-Threonin oder L-Valin-L-Serin als die Peptidkomponente enthält, und wobei das Isodipeptid der Formel (1) ein Isodipeptid, dargestellt durch die Formel (1c), wie in Anspruch 7 definiert, ist.

## Revendications

1. Procédé de préparation d'un peptide contenant un aminoacide présentant un groupe hydroxy sur sa chaîne latérale, dans lequel on utilise un isodipeptide de la formule (1) suivante comme son unité synthétique : 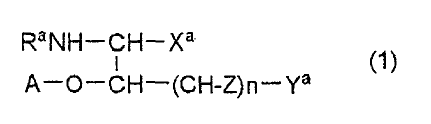 dans laquelle A est un résidu aminoacide N-protégé, R^{a} est un groupe amino protecteur, X^{a} est un groupe carboxyle, un atome d'hydrogène, un groupe alkyle, un groupe aralkyle, un groupe aryle ou un groupe hétéroaryle, Y^{a} est un groupe carboxyle, un atome d'hydrogène ou un groupe alkyle, Z est un atome d'hydrogène ou un groupe alkyle, et n est un nombre entier de 0-3, à condition que l'un ou l'autre de X^{a} et Y^{a} soit un groupe carboxyle.

2. Procédé selon la revendication 1, dans lequel n est égal à 0 ou à 1.

3. Procédé selon la revendication 1, dans lequel dans la formule (1) X^{a} est un groupe carboxyle, Y^{a} est un atome d'hydrogène ou un groupe alkyle et n est égal à 0.

4. Procédé selon la revendication 1, dans lequel dans la formule (1) X^{a} est un atome d'hydrogène, un groupe alkyle, un groupe aralkyle, un groupe aryle ou un groupe hétéroaryle, Y^{a} est un groupe carboxyle et n est égal à 0.

5. Procédé selon la revendication 1, dans lequel le peptide est un peptide contenant de la L-thréonine ou de la L-sérine en tant que constituant de peptide et dans lequel l'isodipeptide de la formule (1) est un isodipeptide représenté par la formule (1a) suivante : 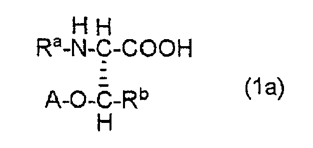 dans laquelle A et R^{a} sont identiques à ceux définis ci-dessus et R^{b} est un atome d'hydrogène ou un groupe méthyle.

6. Procédé selon la revendication 1, dans lequel le peptide est un peptide contenant de la L-valine-L-thréonine ou de la L-valine-L-sérine en tant que constituant de peptide et dans lequel l'isodipeptide de la formule (1) est un isodipeptide représenté par la formule (1b) suivante : 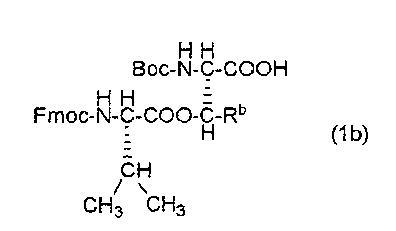 dans laquelle Fmoc est le groupe 9H-fluorèn-9-ylméthoxycarbonyle, Boc est le groupe tert-butoxycarbonyle et R^{b} est identique à celui défini ci-dessus.

7. Procédé selon la revendication 1, dans lequel le peptide est un peptide contenant de la L-valine-L-thréonine ou de la L-valine-L-sérine en tant que constituant de peptide et dans lequel l'isodipeptide de la formule (1) est un isodipeptide représenté par la formule (1c) suivante : 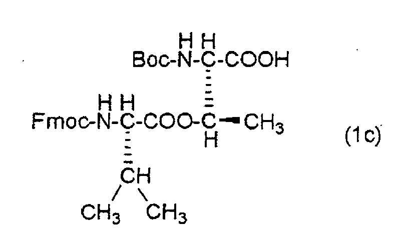 dans laquelle Fmoc et Boc sont identiques à ceux définis ci-dessous.

8. Utilisation d'un isodipeptide de la formule (1) selon l'une quelconque des revendications 1 à 4 en tant qu'unité synthétique dans la préparation d'un peptide contenant un aminoacide présentant un groupe hydroxy sur sa chaîne latérale.

9. Utilisation selon la revendication 8, dans laquelle le peptide est un peptide contenant de la L-thréonine ou de la L-sérine en tant que constituant de peptide et dans laquelle l'isodipeptide de la formule (1) est un isodipeptide représenté par la formule (1a) comme défini dans la revendication 5.

10. Utilisation selon la revendication 8, dans laquelle le peptide est un peptide contenant de la L-valine-L-thréonine ou de la L-valine-L-sérine en tant que constituant de peptide et dans laquelle l'isodipeptide de la formule (1) est un isodipeptide représenté par la formule (1b) comme défini dans la revendication 6.

11. Utilisation selon la revendication 8, dans laquelle le peptide est un peptide contenant de la L-valine-L-thréonine ou de la L-valine-L-sérine en tant que constituant de peptide et dans laquelle l'isodipeptide de la formule (1) est un isodipeptide représenté par la formule (1c) comme défini dans la revendication 7.


### SEQUENCE LISTING

<110> KISO, Yoshiaki
<120> NOVEL ISODIPEPTIDE
<130> P2754 EP
<150> JP 2006-044853
   <151> 2006-02-22
<160> 1
<170> PatentIn version 3.3
<210> 1
   <211> 5
   <212> PRT
   <213> Artificial sequence
<220>
   <221> source
   <223> /note="Description of artificial sequence: synthetic peptide (3a)"
<400> 1
   Val Val Thr Val Val
   1               5